# EUROPEAN PATENT SPECIFICATION

(11) **EP 2 299 261 B1**
(45) Date of publication and mention of the grant of the patent: **10.02.2016**
(21) Application number: 09011896.9
(22) Date of filing: 18.09.2009
(51) Int. Cl.: G01N 27/02, F24C 7/08, G01N 33/02

(54) **Use of a food probe and a method for recognising the type of a food and monitoring a cooking process of a food stuff**
Verwendung einer Nahrungssonde und Verfahren zur Erkennung der Nahrungsart und Überwachung des Kochprozesses von Nahrungsmitteln
Utilisation d'une sonde alimentaire et procédé de reconnaissance du type d'aliment et surveillance d'un procédé de cuisson et aliment

(43) Date of publication of application: 23.03.2011
(73) Proprietor: Electrolux Home Products Corporation N.V., 1130 Brussels (BE)
(72) Inventor: Erbe, Sebastian, 99817 Eisenach (DE); Ruther, Florian, 91628 Steinsfeld (DE)
(74) Representative: Baumgartl, Gerhard Willi

(56) References cited:
- EP-A2- 1 253 423
- DE-A1- 3 621 999
- DE-A1- 3 938 823
- GB-A- 2 298 923
- US-B1- 6 274 850
- US-B2- 6 875 958

## Description

The present invention relates to a method for monitoring a cooking process of food stuff according to the preamble of claim 1. Further, the present invention relates to a use of a food probe for a method for monitoring a cooking process of food stuff according to the preamble of claim 9.

The cooking process of substantially homogenous food stuffs like meat, potatoes and other vegetables, pies and some casseroles is monitored mainly by detecting the core temperature of the food stuff. The user has to know at which core temperatures the food stuff has certain desired properties like colour, tenderness or degree of cooking.

In order to simplify the cooking process for the user, automatic cooking functions are available. Said cooking functions base on an information input from the user and/or measurements of different sensors like a temperature probe. The determination of food properties by measurements allows a reduction of the information input from the user.

The detection of the electrical impedance of the food stuff can provide a lot of information for the automatic cooking functions.

US 2006/0174775 A1 discloses a method and an apparatus for tracing a cooking process. At least two separated electrodes are inserted into the food stuff. The electrical impedance is measure at a certain frequency in order to monitor the cooking process. However, the type of food stuff cannot be recognized.

DE 36 21 999 A1 discloses a method for monitoring a cooking process of food. An elongated food probe is invaded into the food stuff. An electrical impedance of the food stuff is detected in the beginning of the cooking process. The electrical impedance of the food stuff is detected during the further cooking process. The food probe is formed as an elongated rod with at least two electrodes in the front portion of said rod.

It is an object of the present invention to provide a method for monitoring a cooking process, wherein said method allows the recognition of the food, and wherein said food is recognized by very efficient parameters.

This object is achieved by the method for monitoring a cooking process of food stuff according to claim 1.

According to the present invention
- the electrical impedance of the food stuff is detected at two or more frequencies,
- the phase angle of the electrical impedance of the food stuff is calculated from the electrical impedance at two or more frequencies,
- the calculated phase angle of the electrical impedance as function of the frequency is compared with a data base, and
- the type of a food is recognized.

The main idea of the inventive method is the detection of the electrical impedance of the food stuff at two or more frequencies, wherein the phase angle of the electrical impedance of the food stuff are calculated from the electrical impedance. In particular, this additional information allows recognizing the type of a food. The food probe with two arranged electrodes at the one rod is used. Said arranged electrodes at the food probe allow the generation of an electrical field in the core of the food stuff. The food probe is a compact tool and easy to handle.

Preferably, the food probe is invaded into the food stuff in such a way, that the front portion of the rod and the at least two electrodes are arranged within a core of the food stuff.

In particular, the ratio of phase angle of the electrical impedance at two different frequencies is calculated as a function of the temperature of the food stuff.

For example, one of the different frequencies is 50 kHz or 5 kHz. Preferably, the two different frequencies are 50 kHz and 5 kHz.

Further, the first and/or second derivations of the detected and/or calculated parameters may be determined.

The data base may comprise the frequency spectra of the phase angle of the electrical impedance for a plurality of types of food stuff.

Preferably, the data base can be supplemented by the user. Thus, the user can adapt the data base to individual recipes. Such a teach-in function allows the user a possibility to train the cooking oven for recognizing individual recipes, which are not yet in the data base. The object of the present invention is further achieved by the use of a food probe for a method for monitoring a cooking process.

According to the present invention the rod of the used food probe is made of a non-conductive material and the food probe is provided for the above mentioned method for monitoring a cooking process of food stuff.

The main idea of the invention is the use of the food probe for the above mentioned method, wherein the rod of said food probe is made of non-conductive material. The food probe is a compact tool and easy to handle.

In a preferred embodiment of the present invention the food probe comprises a spike arranged at a front end of the rod. The spike allows an easy invading of the food probe into the food stuff.

Further, the food probe may be connected or connectable to voltage supply and a control circuit of a cooking oven.

In particular, the food probe is provided recognizing the type of the food stuff and monitoring a cooking process of said food stuff.

The novel and inventive features believed to be the characteristic of the present invention are set forth in the appended claims.

The invention will be described in further detail with reference to the drawings, in which
- FIG. 1: illustrates a schematic view of a food probe according to a preferred embodiment of the present invention,
- FIG. 2: illustrates a schematic diagram of a phase angle as a function of the frequency for several types of food according to the preferred embodiment of the invention, and
- FIG. 3: illustrates a schematic diagram of a ratio of two phase angles at different frequencies as a function of the temperature according to the preferred embodiment of the invention.

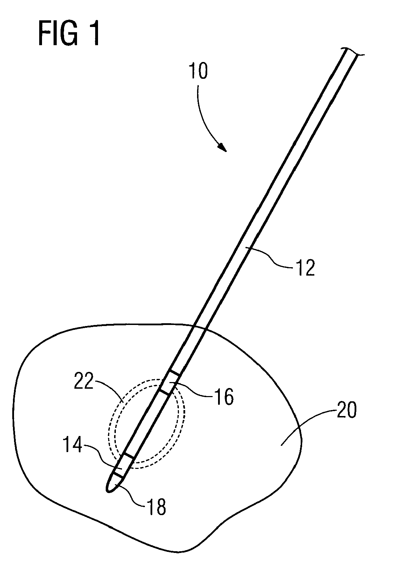

FIG. 1 illustrates a schematic view of a food probe 10 according to a preferred embodiment of the present invention. The food probe 10 is provided for recognizing the type of a food and monitoring a cooking process of a food stuff.

The food probe 10 comprises an elongated rod 12, a first electrode 14, a second electrode 16 and a spike 18. A front portion of the food probe 10 is invaded in a food stuff 20.

The rod 12 is made of a non-conductive material. The first electrode 14 and the second electrode 16 are made of a conductive material. The spike 18 is made of a non-conductive material again.

The spike 18 is arranged at a front end of the rod 10. The spike 18 allows that the food probe 10 can easily be invaded into the food stuff 20.

The first electrode 14 and the second electrode 16 are also arranged within the front portion of the rod 12. The first electrode 14 is arranged besides the spike 18. The second electrode 16 is also arranged within the front portion of the rod 12, but in a predetermined distance from the first electrode 14. Thus, the first electrode 14 and the second electrode 16 are electrically isolated from each other.

When the front portion of the food probe 10 is invaded in the food stuff 20, then the first electrode 14 and the second electrode 16 are also arranged within the food stuff 20.

When a voltage is applied between the first electrode 14 and the second electrode 16, then an electric field 22 is generated within the food stuff 20. Said electric field 22 extends between and in the environment of the first electrode 14 and the second electrode 16.

Preferably, the front portion of the food probe 10 is invaded into the food stuff 20 in such a way, that the electric field 22 is generated within the central portion of the food stuff 20.

The serially arranged electrodes at the food probe 10 allow the generation of the electrical field 22 in the core of the food stuff 20. The food probe 10 is a compact tool and easy to handle.

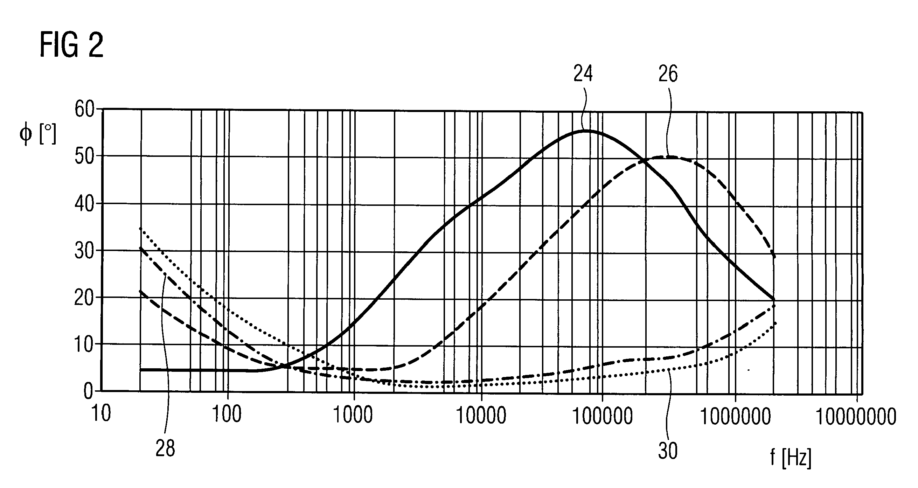

FIG. 2 illustrates a schematic diagram of a phase angle φ as a function of the frequency f for several types of food according to the preferred embodiment of the invention.

A first curve 24 shows the frequency spectrum of the phase angle φ for potatoes. A second curve 26 shows the frequency spectrum of the phase angle φ for cauliflower. A third curve 28 shows the frequency spectrum of the phase angle φ for pork. A fourth curve 30 shows the frequency spectrum of the phase angle φ for the breast of a turkey hen. The spectra of the frequencies in FIG 2 extend from about 10 Hz to about 1 MHz.

FIG. 2 clarifies that different types of food have their own characteristic phase angles φ as function of the frequency f. The frequency spectrum of the phase angle φ can be detected and compared with a data base. Thus, the type of food can be automatically recognized.

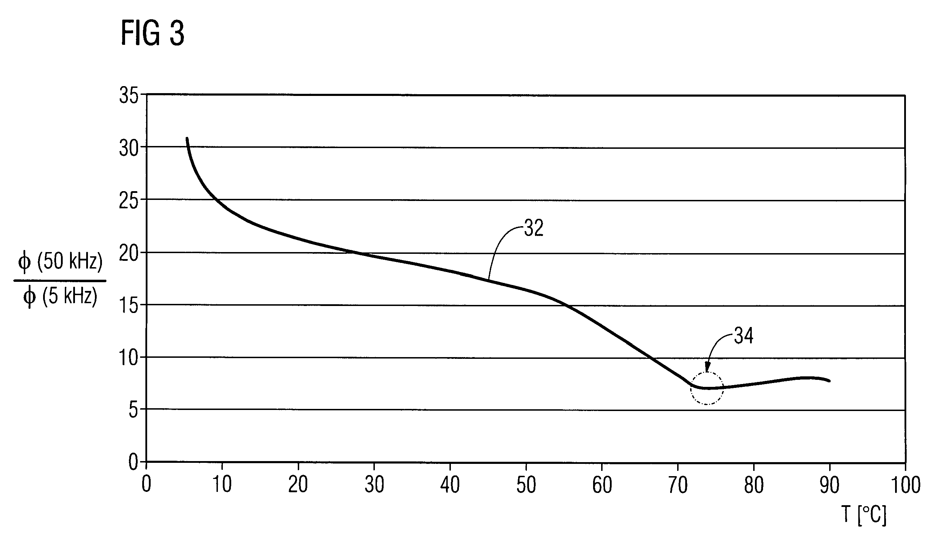

FIG. 3 illustrates a schematic diagram of a ratio of two phase angles φ at different frequencies as a function of the temperature according to the preferred embodiment of the invention. The curve 32 relates to the ratio of the phase angles φ at the frequencies of 50 kHz and 5 kHz. The curve 32 relates to meat.

The spectrum of the temperature in FIG 3 extends from about 10 °C to about 90 °C. At a point 34 the curve 32 has a minimum. At the point 34 all proteins of the food stuff are denaturated, i.e. the meat is fully cooked. The point 34 corresponds with a temperature between 70 °C and 80 °C.

Further parameters like resistances, capacities and specific dielectric constants can be calculated from the detected parameters. Additionally, the first and second derivations of the detected and/or calculated parameters can be determined.

In order to obtain more information the temperature of the food stuff 20 can be detected additionally.

A teach-in function can be implemented. Such a teach-in function allows the user a possibility to train the cooking oven for recognizing individual recipes, which are not yet in the data base.

The present invention can also be embedded in a computer program product, which comprises all the features enabling the implementation of the method described herein. Further, when loaded in a computer system, said computer program product is able to carry out these methods.

Although illustrative embodiments of the present invention have been described herein with reference to the accompanying drawing, it is to be understood that the present invention is not limited to those precise embodiments, and that various other changes and modifications may be affected therein by one skilled in the art without departing from the scope or spirit of the invention. All such changes and modifications are intended to be included within the scope of the invention as defined by the appended claims.

### List of reference numerals

- 10: food probe
- 12: rod
- 14: first electrode
- 16: second electrode
- 18: spike
- 20: food stuff
- 22: electric field
- 24: frequency spectrum of phase angle for potatoes
- 26: frequency spectrum of phase angle for cauliflowers
- 28: frequency spectrum of phase angle for pork
- 30: frequency spectrum of phase angle for turkey
- 32: ratio of phase angle as function of temperature
- 34: point where all proteins are denaturated

- φ: phase angle
- f: frequency
- T: temperature

## Claims

1. A method for monitoring a cooking process of food stuff (20) comprising the steps of:
- invading an elongated food probe (10) into the food stuff (20),
- detecting an electrical impedance of the food stuff (20) in the beginning of the cooking process, and
- detecting the electrical impedance of the food stuff (20) during the further cooking process, wherein
- the food probe (10) is formed as an elongated rod (12) with at least two electrodes (14, 16) in the front portion of said rod (12).
**characterized in, that**
- the electrical impedance of the food stuff (20) is detected at two or more frequencies (f),
- the phase angle (Φ) of the electrical impedance of the food stuff (20) is calculated from the electrical impedance at two or more frequencies (f),
- the calculated phase angle (Φ) of the electrical impedance as function of the frequency (f) is compared with a data base, and
- the type of a food is recognized.

2. The method according to claim 1, **characterized in, that** the food probe (10) is invaded into the food stuff (20) in such a way, that the front portion of the rod (12) and the at least two electrodes (14, 16) are arranged within a core of the food stuff (20).

3. The method according to claim 1 or 2,
**characterized in, that**
the ratio of phase angle (φ) of the electrical impedance at two different frequencies is calculated as a function of the temperature of the food stuff (20).

4. The method according to claim 3,
**characterized in, that**
one of the two different frequencies is 50 kHz.

5. The method according to claim 3 or 4,
**characterized in, that**
one of the two different frequencies is 5 kHz.

6. The method according to any one of the preceding claims,
**characterized in, that**
the first and/or second derivations of the detected and/or calculated parameters are determined.

7. The method according to any one of the preceding claims,
**characterized in, that**
the data base comprises frequency spectra of the phase angle (φ) of the electrical impedance for a plurality of types of food stuff (20).

8. The method according to any one of the preceding claims,
**characterized in, that**
the data base can be supplemented by the user.

9. A use of a food probe (10) for a method for monitoring a cooking process of food stuff (20), wherein said food probe (10) comprises:
- an elongated rod (12),
- a front portion of the elongated rod (12), which front portion is provided for invading into the food stuff (20),
- a first electrode (14) made of a conductive material and arranged at the front portion of the rod (12), and
- a second electrode (16) made of a conductive material and arranged at the front portion of the rod (12) in a predetermined distance from the first electrode (14), wherein
- the first electrode (14) and the second electrode (16) are arranged serially along the longitudinal axis of the rod (12), and wherein
- a voltage can be applied between the first electrode (14) and the second electrode (16), so that an electrical field (22) is generated between and in the environment of the first electrode (14) and the second electrode (16).
**characterized in, that**
the rod (12) is made of a non-conductive material and the food probe (10) is provided for a method according to any one of the claims 1 to 8.

10. The use of the food probe according to claim 9,
**characterized in, that**
the food probe (10) comprises a spike (18) arranged at a front end of the rod (12).

11. The use of the food probe according to claim 9 or 10,
**characterized in, that**
the food probe (10) is connected or connectable to voltage supply and a control circuit of a cooking oven.

12. The use of the food probe according to any one of the claims 9 to 11,
**characterized in, that**
the food probe (10) is provided recognizing the type of the food stuff and monitoring a cooking process of said food stuff (20).

## Patentansprüche

1. Verfahren zum Überwachen eines Kochvorgangs eines Lebensmittels (20), das die folgenden Schritte umfasst:
- Einstecken eines länglichen Lebensmittel-Messfühlers (10) in das Lebensmittel (20),
- Detektieren einer elektrischen Impedanz des Lebensmittels (20) zu Beginn des Kochvorgangs, und
- Detektieren der elektrischen Impedanz des Lebensmittels (20) während des weiteren Kochvorgangs, wobei
- der Lebensmittel-Messfühler (10) als ein länglicher Stab (12) mit wenigstens zwei Elektroden (14, 16) in dem vorderen Abschnitt des Stabs (12) ausgebildet ist;
**dadurch gekennzeichnet, dass**
- die elektrische Impedanz des Lebensmittels (20) bei zwei oder mehr Frequenzen (f) detektiert wird,
- der Phasenwinkel (φ) der elektrischen Impedanz des Lebensmittels (20) aus der elektrischen Impedanz bei zwei oder mehr Frequenzen (f) berechnet wird,
- der berechnete Phasenwinkel (φ) der elektrischen Impedanz in Abhängigkeit von der Frequenz (f) mit einer Datenbasis verglichen wird, und
- die Art eines Lebensmittels erkannt wird.

2. Verfahren nach Anspruch 1, **dadurch gekennzeichnet, dass**
der Lebensmittel-Messfühler (10) so in das Lebensmittel (20) eindringt, dass der vordere Abschnitt des Stabs (12) und die wenigstens zwei Elektroden (14, 16) in einem Kern des Lebensmittels (20) angeordnet sind.

3. Verfahren nach Anspruch 1 oder 2,
**dadurch gekennzeichnet, dass**
das Verhältnis des Phasenwinkels (Φ) der elektrischen Impedanz bei zwei verschiedenen Frequenzen in Abhängigkeit von der Temperatur des Lebensmittels (20) berechnet wird.

4. Verfahren nach Anspruch 3,
**dadurch gekennzeichnet, dass**
eine der zwei verschiedenen Frequenzen 50 kHz beträgt.

5. Verfahren nach Anspruch 3 oder 4,
**dadurch gekennzeichnet, dass**
eine der zwei verschiedenen Frequenzen 5 kHz beträgt.

6. Verfahren nach einem der vorhergehenden Ansprüche,
**dadurch gekennzeichnet, dass**
die erste bzw. zweite Ableitung der detektierten bzw. berechneten Parameter bestimmt wird.

7. Verfahren nach einem der vorhergehenden Ansprüche,
**dadurch gekennzeichnet, dass**
die Datenbasis Frequenzspektren des Phasenwinkels (Φ) der elektrischen Impedanz für mehrere Arten von Lebensmitteln (20) umfasst.

8. Verfahren nach einem der vorhergehenden Ansprüche,
**dadurch gekennzeichnet, dass**
die Datenbasis durch den Benutzer ergänzt werden kann.

9. Verwendung eines Lebensmittel-Messfühlers (10) für ein Verfahren zum Überwachen eines Kochvorgangs eines Lebensmittels (20), wobei der Lebensmittel-Messfühler (10) Folgendes umfasst:
- einen länglichen Stab (12),
- einen vorderen Abschnitt des länglichen Stabs (12), wobei der vordere Abschnitt zum Einstecken in das Lebensmittel (20) vorgesehen ist,
- eine erste Elektrode (14), die aus einem leitfähigen Material hergestellt ist und an dem vorderen Abschnitt des Stabs (12) angeordnet ist, und
- eine zweite Elektrode (16), die aus einem leitfähigen Material hergestellt ist und an dem vorderen Abschnitt des Stabs (12) in einem vorher festgelegten Abstand von der ersten Elektrode (14) angeordnet ist, wobei
- die erste Elektrode (14) und die zweite Elektrode (16) längs der Längsachse des Stabs (12) hintereinander angeordnet sind, und wobei
- eine Spannung zwischen der ersten Elektrode (14) und der zweiten Elektrode (16) angelegt werden kann, so dass zwischen der ersten Elektrode (14) und der zweiten Elektrode (16) und in deren Umgebung ein elektrisches Feld (22) erzeugt wird;
**dadurch gekennzeichnet, dass**
der Stab (12) aus einem nicht leitfähigen Material hergestellt ist und dass der Lebensmittel-Messfühler (10) für ein Verfahren nach einem der Ansprüche 1 bis 8 vorgesehen ist.

10. Verwendung des Lebensmittel-Messfühlers nach Anspruch 9,
**dadurch gekennzeichnet, dass**
der Lebensmittel-Messfühler (10) eine Spitze (18) umfasst, die an einem vorderen Ende des Stabs (12) angeordnet ist.

11. Verwendung des Lebensmittel-Messfühlers nach Anspruch 9 oder 10,
**dadurch gekennzeichnet, dass**
der Lebensmittel-Messfühler (10) mit einer Spannungsquelle und einer Steuerungsschaltung eines Backofens verbunden ist oder verbunden werden kann.

12. Verwendung des Lebensmittel-Messfühlers nach einem der Ansprüche 9 bis 11,
**dadurch gekennzeichnet, dass**
der Lebensmittel-Messfühler (10) zum Erkennen der Art des Lebensmittels und zum Überwachen eines Kochvorgangs des Lebensmittels (20) vorgesehen ist.

## Revendications

1. Procédé de surveillance d'une opération de cuisson d'un aliment (20), le procédé comportant les étapes qui consistent à :
introduire une sonde alimentaire allongée (10) dans l'aliment (20),
détecter l'impédance électrique de l'aliment (20) au début de l'opération de cuisson et
détecter l'impédance électrique de l'aliment (20) pendant la poursuite de l'opération de cuisson,
la sonde alimentaire (10) étant configurée comme barre allongée (12) présentant au moins deux électrodes (14, 16) dans la partie avant de ladite barre (12), **caractérisé en ce que**
l'impédance électrique de l'aliment (20) est détectée à deux ou plusieurs fréquences (f),
le déphasage (φ) de l'impédance électrique de l'aliment (20) est calculé à partir de l'impédance électrique à deux ou plusieurs fréquences (f),
le déphasage calculé (φ) de l'impédance électrique en fonction de la fréquence (f) est comparé avec une base de données et
le type d'aliment est reconnu.

2. Procédé selon la revendication 1, **caractérisé en ce que** la sonde alimentaire (10) est introduite dans l'aliment (20) de telle sorte que la partie avant de la barre (12) et les deux ou plusieurs électrodes (14, 16) soient agencées au coeur de l'aliment (20).

3. Procédé selon les revendications 1 ou 2, **caractérisé en ce que** le rapport entre le déphasage (φ) de l'impédance électrique à deux fréquences différentes est calculé en fonction de la température de l'aliment (20).

4. Procédé selon la revendication 3, **caractérisé en ce que** l'une des deux différentes fréquences est de 50 kHz.

5. Procédé selon les revendications 3 ou 4, **caractérisé en ce que** l'une des deux fréquences différentes est de 5 kHz.

6. Procédé selon l'une quelconque des revendications précédentes, **caractérisé en ce que** la première et/ou la deuxième dérivée des paramètres détectés et/ou calculés sont déterminées.

7. Procédé selon l'une quelconque des revendications précédentes, **caractérisé en ce que** la base de données comprend des spectres de fréquence du déphasage (φ) de l'impédance électrique pour plusieurs types d'aliments (20).

8. Procédé selon l'une quelconque des revendications précédentes, **caractérisé en ce que** la base de données peut être complétée par l'utilisateur.

9. Utilisation d'une sonde alimentaire (10) dans un procédé de surveillance d'une opération de cuisson d'un aliment (20), ladite sonde alimentaire (10) comprenant :
une barre allongée (12),
une partie avant de la barre allongée (12), laquelle partie avant est prévue pour être introduite dans l'aliment (20),
une première électrode (14) constituée d'un matériau conducteur et agencée sur la partie avant de la barre (12) et
une deuxième électrode (16) constituée d'un matériau conducteur et agencée sur la partie avant de la barre (12) à une distance prédéterminée de la première électrode (14),
la première électrode (14) et la deuxième électrode (16) étant agencées en série selon l'axe longitudinal de la barre (12) et
une tension pouvant être appliquée entre la première électrode (14) et la deuxième électrode (16) de manière à produire un champ électrique (22) entre la première électrode (14) et la deuxième électrode (16) dans l'environnement de ces dernières,
**caractérisée en ce que**
la barre (12) est constituée d'un matériau non conducteur et la sonde alimentaire (10) est prévue pour un procédé selon l'une quelconque des revendications 1 à 8.

10. Utilisation de la sonde alimentaire selon la revendication 9, **caractérisée en ce que** la sonde alimentaire (10) comprend une pointe (18) agencée à l'extrémité avant de la barre (12).

11. Utilisation de la sonde alimentaire selon les revendications 9 ou 10, **caractérisée en ce que** la sonde alimentaire (10) est raccordée ou peut être raccordée à une alimentation en tension et à un circuit de commande d'un four de cuisson.

12. Utilisation de la sonde alimentaire selon l'une quelconque des revendications 9 à 11, **caractérisée en ce que** la sonde alimentaire (10) est prévue pour reconnaître le type d'aliment et pour surveiller l'opération de cuisson dudit aliment (20).
